# EUROPEAN PATENT SPECIFICATION

(11) **EP 1 887 866 B1**
(45) Date of publication and mention of the grant of the patent: **06.12.2017**
(21) Application number: 06784410.0
(22) Date of filing: 15.05.2006
(51) Int. Cl.: A01N 47/02, A01N 43/56, A01N 43/90, A61K 9/00, A01P 7/00, A01P 5/00, A61P 33/00, A61P 33/10, A61P 33/14

(54) **SPOT-ON FORMULATIONS FOR COMBATING PARASITES**
SPOT-ON FORMULIERUNGEN ZUR PARASITENBEKÄMPFUNG
FORMULATIONS DE TRAITEMENT CUTANE LOCALISE CONTRE LES PARASITES

(30) Priority: 16.05.2005 US 55234
(43) Date of publication of application: 20.02.2008
(73) Proprietor: Merial, Inc., Georgia 30096 (US)
(72) Inventor: BOECKH, Albert, Cumming, GA 30041 (US); SOLL, Mark, Alpharetta, GA 30005 (US); JEANNIN, Philippe, F-69110 Ste Foy Les Lyon (FR)
(74) Representative: D Young & Co LLP
(86) International application number: PCT/US2006/018657
(87) International publication number: WO 2007/018659

(56) References cited:
- EP-A- 1 668 984
- EP-A1- 0 679 650
- EP-A1- 1 449 435
- EP-A2- 1 066 854
- GB-A- 2 275 193
- US-A- 6 010 710
- US-B1- 6 482 425
- BISHOP B F ET AL: "Selamectin: A novel broad-spectrum endectocide for dogs and cats" VETERINARY PARASITOLOGY, ELSEVIER SCIENCE, AMSTERDAM, NL, vol. 91, no. 3-4, 2000, pages 163-176, XP002202198 ISSN: 0304-4017

## Description

### FIELD OF THE INVENTION

This invention relates to spot-on formulations for combating parasites in birds and mammals. In particular, this invention provides for spot-on formulations comprising a composition comprising a 1-N-phenylpyrazole derivative and a macrolide anthelmintic or antiparasitic agent, and a pharmaceutically or veterinary acceptable liquid carrier vehicle. This invention also provides for to an improved method for eradicating, controlling, and preventing parasite infestation in birds and mammals.

### BACKGROUND OF THE INVENTION

Animals such as mammals and birds are often susceptible to parasite infestations. These parasites may be ectoparasites, such as insects, and endoparasites such as filariae and worms.

Domesticated animal, such as cats and dogs, are often infested with one or more of the following ectoparasites:
- cat and dog fleas (*Ctenocephalides felis, Ctenocephalides* sp. and the like),
- ticks (*Rhipicephalus* sp., *Ixodes* sp., *Dermacentor* sp., *Amblyoma* sp. and the like), and
- mites (*Demodex* sp., *Sarcoptes* sp., *Otodectes* sp. and the like),
- lice (*Trichodectes* sp., *Cheyletiella* sp., *Lignonathus* sp., and the like),
- mosquitoes (*Aedes* sp., *Culex* sp., *Anopheles* sp., and the like) and
- flies (*Hematobia* sp., *Musca* sp., *Stomoxys* sp., *Dermatobia* sp., *Cochlyomia* sp., and the like).

Fleas are a particular problem because not only do they adversely affect the health of the animal or human, but they also cause a great deal of psychological stress. Moreover, fleas are also vectors of pathogenic agents in animals, such as dog tapeworm (*Dipylidium caninum*), and humans.

Similarly, ticks are also harmful to the physical and psychological health of the animal or human. However, the most serious problem associated with ticks is that they are the vector of pathogenic agents, agents which cause diseases in both humans and animal. Major diseases which are caused by ticks include borrelioses (Lyme disease caused by *Borrelia burgdorferi*), babesioses (or piroplasmoses caused by *Babesia* sp.) and rickettsioses (also known as Rocky Mountain spotted fever). Ticks also release toxins which cause inflammation or paralysis in the host. Occasionally, these toxins are fatal to the host.

Moreover, mites and lice are particularly difficult to combat since there are very few active substances which act on these parasites and they require frequent treatment.

Likewise, farm animals are also susceptible to parasite infestations. For example, cattle are affected by a large number of parasites. A parasite which is very prevalent among farm animals is a tick genus *Boophilus,* especially those of the species microplus (cattle tick), decoloratus and anulatus. Ticks, such as *Boophilus microplus,* are particularly difficult to control because they live in the pasture where the farm animals graze. Other important parasites of cattle and sheep are listed as follows in order of decreasing importance:
- myiases such as *Dermatobia hominis* (known as Berne in Brazil) and *Cochlyomia hominivorax* (greenbottle); sheep myiases such as *Lucilia sericata, Lucilia cuprina* (known as blowfly strike in Australia, New Zealand and South Africa). These are flies whose larva constitutes the animal parasite;
- flies proper, namely those whose adult constitutes the parasite, such as *Haematobia irritans* (horn fly);
- lice such as *Linognathus vitulorum,* etc.; and
- mites such as *Sarcoptes scabiei* and *Psoroptes ovis.*
The above list is not exhaustive and other ectoparasites are well known in the art to be harmful to animals and humans. These include, for example migrating dipterous larvae.

Animals and humans also suffer from endoparasitical infections including, for example, helminthiasis which is most frequently caused by a group of parasitic worms described as nematodes or heartworms or roundworms. These parasites cause severe economic losses in pigs, sheep, horses, and cattle as well as affecting domestic animals and poultry. Other parasites which occur in the gastrointestinal tract of animals and humans include *Ancylostoma, Necator, Ascaris, Strongyloides, Trichinella, Capillaria, Toxocara, Toxascaris, Trichuris*, *Enterobius* and parasites which are found in the blood or other tissues and organs such as filarial worms and the extra intestinal stages of *Strogyloides*, *Toxocara* and *Trichinella*.

Many insecticides exist in the art for treating parasites. These insecticides vary in their effectiveness to a particular parasite as well as their cost. However the results of these insecticides is not always satisfactory because of, for example, the development of resistance by the parasite to the therapeutic agent, as is the case, for example, with carbamates, organophosphorus compounds and pyrethroids. Moreover, there is at the present time no truly effective method for controlling both ticks and helminths and less still an effective way of controlling the set of parasites indicated above. Thus, there is a need in the art for more effective antiparasitic formulation treatment and protection of animal, *e.g*. mammals, fish and birds for a wide range of parasites. Moreover, there is a need in the art for antiparasitic formulation which is easy to use on any type of domestic animal, irrespective of its size and the nature of its coat and which do not need to be sprinkled over the entire body of the mammal, fish or bird.

A new family of insecticides based on 1-N-phenylpyrazoles is described in Patents EP-A-295,217 and EP-A-352,944. The compounds of the families defined in these patents are extremely active and one of these compounds, 1-[2,6-Cl₂-4-CF₃ phenyl]-3-CN-4-[SO-CF₃]-5-NH₂ pyrazole, or fipronil, is particularly effective, not only against crop parasites but also against ectoparasites of mammals and birds. Fipronil is particularly, but not exclusively, effective against fleas and ticks.

Endectocidal compounds, which exhibit a degree of activity against a wide range endoparasites, are known in the art. These compounds possess a macrocyclic lactone ring and are known in the art to be particularly effective against ectoparasites, including lice, blowflies, flies, mosquitoes, mites, migrating dipterous larvae, and ticks, as well as endoparasites, such as nematodes, heartworms and roundworms. Compounds of this group include avermectins, milbemycin, and derivatives of these compounds, for example, abamectin, doramectin, emamectin, eprinomectin, ivermectin, latidectin, lepimectin, milbemectin, moxidectin or selamectin. Such substances are described, for example, in U.S. Patents 3,950,360; 4,199,569; 4,879,749; and 5,268,710.

While it is known in the art that it is sometimes possible to combine various parasiticides in order to broaden the antiparasitical spectrum, it is not possible to predict, *a priori,* which combinations will work for a particular animal or disease state. For this reason, the results of various combinations is not always successful and there is a need in the art for more effective formulations which may be easily administered to the animal. The effectiveness of formulations comprising 1-N-phenylpyrazole derivatives and macrolide lactone anthelmintic or parasitic agents, such as avermectins, ivermectins and milbemycin, against an endoparasite or an ectoparasite in a specific host is especially difficult to predict because of the numerous and complex host-parasite interactions.

Patent application AU-A-16 427/95 very broadly mentions the combination of a substituted 1-N-pyrazole derivatives with an avermectin, ivermectin or moxidectin in a discussion involving among a very large number of insecticides or parasiticides of various types, including fipronil. However, this patent application does not provide specific guidance to the skilled artisan on how to formulate a 1-N-pyrazole derivative with an avermectin or milbemycin type compound, let alone how to formulate a spot-on composition comprising these compounds. Moreover, the application does not indicate which specific parasites are susceptible to what specific combination.

US6482425 discloses the present 1-N-pyrazole compounds of formula (I), including fipronil, in combination with various macrocyclic lactones, such as abamectin, avermectin and doramectin. Various methods of formulating antiparasitical formulations are known in the art. These include oral formulations, baits, dietary supplements, powders, shampoos, etc. Formulations for localized topical applications of antiparasitical formulations are also known in the art. For example, pour-on solutions comprising 1-N-phenylpyrazoles, such as fipronil, are known in the art and are described in WO9736486. Other methods of formulating antiparasitic agents include spot-on formulations.

Spot-on formulations are well known techniques for topically delivering an antiparasitic agent to a limited area of the host. For example, U.S. Patent 5,045,536 describes such formulations for ectoparasites. Moreover, it is generally known in the art to formulate avermectin and milbemycin derivatives as spot-on formulations. See, e.g. U.S. Patent 5,045,536; EP 677,054; U.S. Patent 5,733,877; U.S. Patent 5,677,332; U.S. Patent 5,556,868; and U.S. Patent 5,723,488. However, as discussed in U.S. Patent 5,045,536, a large number of solvent systems described in the art provide formulations for localized topical application which cause irritancy or toxicity to the host. Hence, there is a need in the art both for more effect and less irritant or toxic formulations. Thus, there is a need in the art for a spot-on formulation which is effect against a wide range of endoparasites and ectoparasites in birds and mammals.

### BRIEF DESCRIPTION OF THE DRAWING

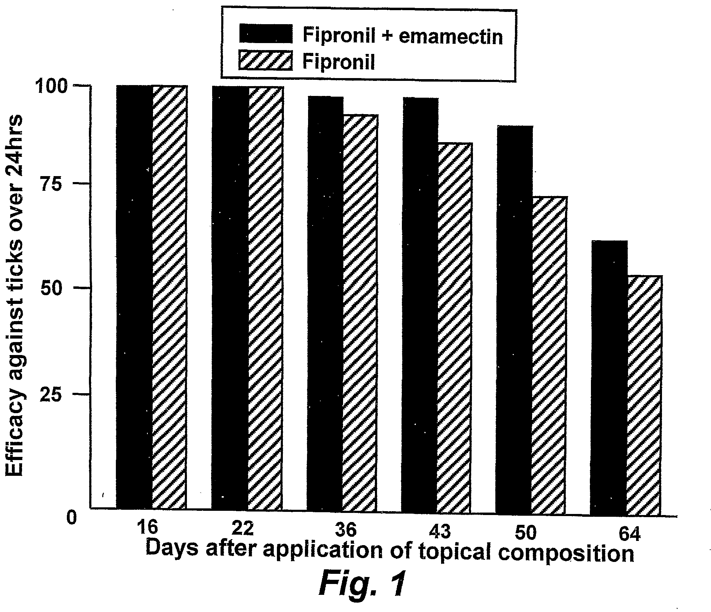

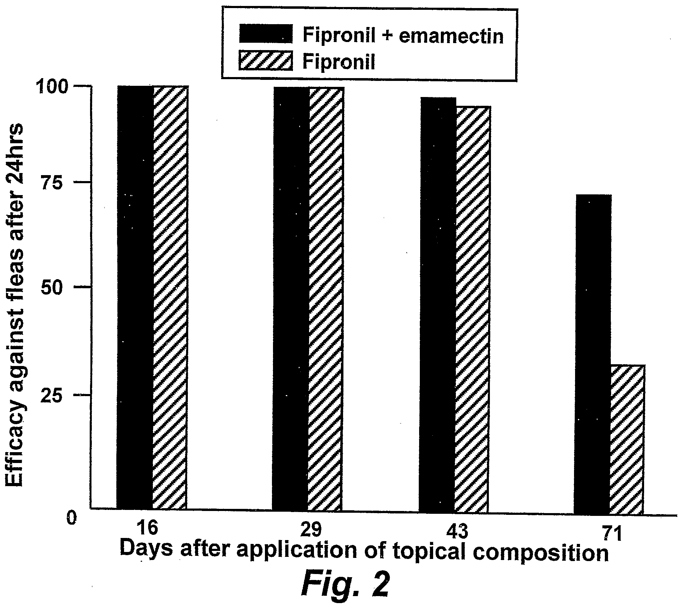

Fig. 1 compares the efficacy against ticks in dogs for the topical application of a topical composition comprising fipronil against the efficacy of a topical application comprising fipronil and emamectin. Fig. 2 compares the efficacy against fleas in dogs for the topical application of a topical composition comprising fipronil against the efficacy of a topical application comprising fipronil and emamectin.

### SUMMARY OF THE INVENTION

The invention provides for spot-on formulations for the treatment or prophylaxis of parasites of mammals and birds, and in particular, cats, dogs, horses, chickens, sheep and cattle with the aim of ridding these hosts of all the parasites commonly encountered by birds and mammals. Disclosed is the effective and lasting destruction of ectoparasites, such as fleas, ticks, mites, *e.g.* itch mites, mosquitoes, flies and lice, and of endoparasites, nematodes, such as filariae, heartworms and roundworms of the digestive tract of animals and humans.

In particular this invention provides a spot-on formulation for the treatment or prophylaxis of parasite infestation in mammals or birds which comprises
(1) a composition comprising
   (A) an effective amount of (1) 1-[2,6-Cl₂-4-CF₃ phenyl]-3-CN-4-[SO-CF₃]-5-NH₂ pyrazole; and
   (B) an effective amount of emamectin or a salt thereof;
(2) a pharmaceutically or veterinary acceptable liquid carrier vehicle wherein the liquid carrier vehicle comprises a solvent and a cosolvent wherein the solvent is selected from the group consisting of acetone, acetonitrile, benzyl alcohol, butyl diglycol, dimethylacetamide, dimethylformamide, dipropylene glycol n-butyl ether, ethanol, isopropanol, methanol, ethylene glycol monoethyl ether, ethylene glycol monomethyl ether, monomethylaceamide, dipropylene glycol monomethyl ether, liquid polyoxyethylene glycols, propylene glycol, 2-pyrrolidone, diethylene glycol monoethyl ether, ethylene glycol, diethyl phthalate, and a mixture of at least two of these solvents and the cosolvent is selected from the group consisting of absolute ethanol, isopropanol or methanol;
(3) a crystallization inhibitor, wherein the crystallization inhibitor is selected from the group consisting of an anionic surfactant, a cationic surfactant, a non-ionic surfactant, an amine salt, an amphoteric surfactant, polyvinylpyrrolidone, polyvinyl alcohols, copolymers of vinyl acetate and vinylpyrrolidone, polyethylene glycols, benzyl alcohol, mannitol, glycerol, sorbitol, polyoxyethylenated sorbitan esters; lecithin, sodium carboxymethylcellulose, and acrylic derivatives, or a mixture of these crystallization inhibitors.

According to another aspect of the present invention there is provided a spot-on composition, which comprises, in a veterinarily acceptable vehicle, an amount parasitically effective of at least one compound (A), and an amount parasitically effective of at least one compound (B), wherein:
compound (A) is of the formula (I) 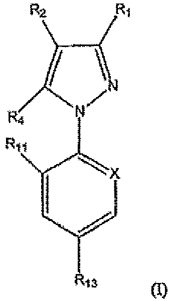
in which:
   R₁ is CN;
   R₂ is S(O)ₙR₃;
   R₃ is haloalkyl;
   R₄ represents NH₂;
   R₁₁ represents halogen atom;
   R₁₃ represents haloalkyl;
   n represents an integer equal to 0, 1 or 2;
   X represents a radical C-R₁₂;
   R₁₂ represents a halogen atom;
and
compound (B) is an endectocidal parasiticide which is emamectin, or a salt thereof; and,
the vehicle is for a localized cutaneous application to the animal between the shoulders and contains an organic solvent, an organic cosolvent and/or a crystallization inhibitor wherein:
   the crystallization inhibitor is selected from the group consisting of polyvinylpyrrolidone, copolymers of vinyl acetate and vinylpyrrolidone, polyoxyethylenated sorbitan esters and mixtures thereof;
   the organic solvent comprises acetone, ethyl acetate, methanol, ethanol, isopropanol, dimethylformamide, dicholoromethane or diethyl glycol monoethyl ether; said solvent optionally supplemented by C₈-C₁₀ caprylic/capric triglyceride, oleic acid or propylene glycol; and
   the organic cosolvent selected from the group consisting of ethanol, isopropanol, and methanol;
   for the use in combating parasites of a cat or dog comprising localized cutaneous application to the cat or dog, between the shoulders, at a frequency not greater than monthly, whereby there is a prolonged relase of compound (A) in or on the body of the cat or dog and there is a measurable plasma level of compound (B) in the cat or dog.

According to a further aspect of the present invention there is provided a use of a spot-on composition, which comprises, an amount parasitically effective of at least one compound (A), and an amount parasitically effective of at least one compound (B), wherein:
compound (A) is of the formula (I) 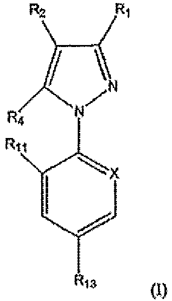
in which:
   R₁ is CN;
   R₂ is S(O)ₙR₃;
   R₃ is haloalkyl;
   R₄ represents NH₂;
   R₁₁ represents halogen atom;
   R₁₃ represents haloalkyl;
   n represents an integer equal to 0, 1 or 2;
   X represents a radical C-R₁₂;
   R₁₂ represents a halogen atom;
and
compound (B) is an endectocidal parasiticide which is emamectin, or a salt thereof,
for the manufacture of a medicament which comprises a veterinarily acceptable vehicle for combating parasites of a cat or dog comprising localized cutaneous application to the cat or dog, between the shoulders, at a frequency not greater than monthly, wherein the vehicle is for a localized cutaneous application to the animal between the shoulders and contains an organic solvent, an organic cosolvent and/or a crystallisation inhibitor wherein:
   the crystallisation inhibitor selected from the group consisting of polyvinylpyrrolidone, copolymers of vinyl acetate and vinylpyrrolidone, polyoxyethylenated sorbitan esters and mixtures thereof;
   the organic solvent comprises acetone, ethyl acetate, methanol, ethanol, isopropanol, dimethylformamide, dichloromethane or diethyl glycol monomethyl ether; said solvent optionally supplemented by C₈-C₁₀ caprylic/capric triglyceride, oleic acid or propylene glycol; and
   the organic cosolvent is selected from the group consisting of ethanol, isopropanol, and methanol;
   whereby there is a prolonged release of compound (A) in or on the body of the cat or dog and there is a measurable plasma level of compound (B) in the cat or dog.
Also disclosed is an easy method of treating parasitic infestations or the prophylaxis of parasite infestations in mammals or birds which comprises topically applying to said mammal or bird an effective amount of a formulation according to the present invention. Also disclosed are spot-on formulations comprising a combination comprising a compound of formula (I) and a macrocyclic lactone which exhibit synergistic activity against parasites when compared to formulations which contain only one class of therapeutic agent.

This invention further provides for formulations which, when applied locally, will diffuse over the entire body of the host and then dry, without crystallizing, and which do not affect the appearance of the coat after drying by, for example, leaving crystals or making the coat sticky. This has the further advantage in animals which groom themselves of not being orally ingested, where the therapeutic agent might not be well tolerated orally or might interact with other therapeutic agents.

The very high effectiveness of the method and of the formulations according to the invention provides not only for a high instantaneous effectiveness but also for an effectiveness of very long duration after the treatment of the animal.

These and other embodiments are disclosed in following Detailed Description.

### DETAILED DESCRIPTION

This invention provides for a spot-on formulation for the treatment or prophylaxis of parasite infestation in mammals or birds which comprises
(1) a composition comprising
   (A) an effective amount of (1) 1-[2,6-Cl₂-4-CF₃ phenyl]-3-CN-4-[SO-CF₃]-5-NH₂ pyrazole; and
   (B) an effective amount of emamectin or a salt thereof;
(2) a pharmaceutically or veterinary acceptable liquid carrier vehicle wherein the liquid carrier vehicle comprises a solvent and a cosolvent wherein the solvent is selected from the group consisting of acetone, acetonitrile, benzyl alcohol, butyl diglycol, dimethylacetamide, dimethylformamide, dipropylene glycol n-butyl ether, ethanol, isopropanol, methanol, ethylene glycol monoethyl ether, ethylene glycol monomethyl ether, monomethylaceamide, dipropylene glycol monomethyl ether, liquid polyoxyethylene glycols, propylene glycol, 2-pyrrolidone, diethylene glycol monoethyl ether, ethylene glycol, diethyl phthalate, and a mixture of at least two of these solvents and the cosolvent is selected from the group consisting of absolute ethanol, isopropanol or methanol;
(3) a crystallization inhibitor, wherein the crystallization inhibitor is selected from the group consisting of an anionic surfactant, a cationic surfactant, a non-ionic surfactant, an amine salt, an amphoteric surfactant, polyvinylpyrrolidone, polyvinyl alcohols, copolymers of vinyl acetate and vinylpyrrolidone, polyethylene glycols, benzyl alcohol, mannitol, glycerol, sorbitol, polyoxyethylenated sorbitan esters; lecithin, sodium carboxymethylcellulose, and acrylic derivatives, or a mixture of these crystallization inhibitors.

Preferred are spot-on compositions for use in the invention, wherein the composition comprises:
(A) 1-[2,6-Cl₂-4-CF₃phenyl]-3-CN-4-[SO-CF3]-5-NH₂pyrazole; and
(B) an effective amount of emamectin, and a salt/salts thereof

The phenylpyrazoles ("compound A") as a class are known in the art and are described, for example, in copending applications USSN 07/719,942; 08/933,016; 09/174,598; 08/863,182; and 08/863,692, as well as in U.S. Patent No. 5,576,429; U.S. Patent No. 5,122,530, and EP 295 177.
This class of insecticides is known to possess excellent activity against insects. such as ticks and fleas.
Emamectin has the following structure: 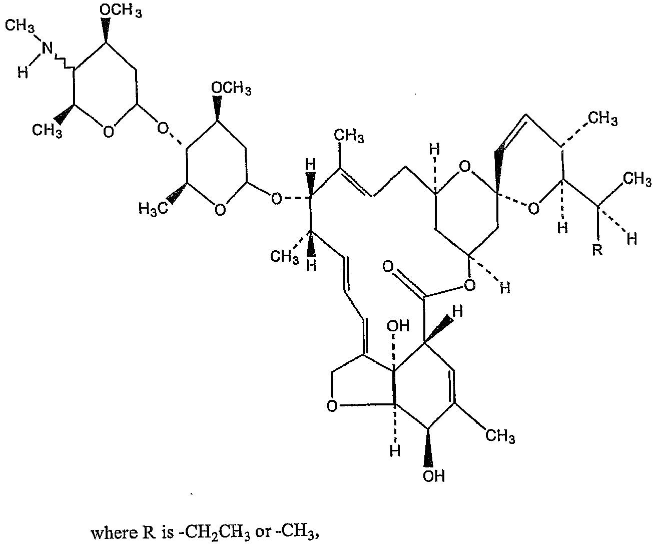 or a salt of this compound. These compounds are described in U.S. Patent Nos. 4,874,749 or 5,288,710.
A compound of formula (I) which is very particularly preferred in the invention is 1-[2,6-Cl₂-4-CF3phenyl]-3-CN-4-[SO-CF₃]-5-NH₂pyrazole or fipronil.

More generally, compounds (A) are phenylpyrazoles and N-arylpyrazoles, and reference is made to, for example, U.S. patent No. 5,567,429, U.S. Patent No. 5,122,530, EP 295,117, and EP 846,686 A1 (or Banks GB 9,625,045, filed November 30, 1996 also believed to be equivalent to USSN 309,229, filed November 17, 1997).

Compounds of formula (I) can be prepared according to one or other of the processes described in Patent Applications WO 87/3781, 93/6089 and 94/21606 or European Patent Application 295,117 or any other process coming within the competence of a person skilled in the art who is an expert in chemical synthesis. For the chemical preparation of the products of the invention, a person skilled in the art is regarded as having at his disposal, *inter alia,* the entire contents of "Chemical Abstracts" and of the documents which are cited therein.

Administration of the inventive formulation may be intermittent in time and may be administered daily, weekly, biweekly, monthly, bimonthly, quarterly, or even for longer durations of time. The time period between treatments depends upon factors such as the parasite(s) being treated, the degree of infestation, the type of mammal or bird and the environment where it resides. It is well within the skill level of the practitioner to determine a specific administration period for a particular situation. This invention contemplates a method for permanently combating a parasite in an environment in which the animal is subjected to strong parasitic pressure where the administration is at a frequency far below a daily administration in this case. For example, it is preferable for the treatment according to the invention to be carried out monthly on dogs and on cats.

Spot-on formulations may be prepared by dissolving the active ingredients into the pharmaceutically or veterinary acceptable vehicle. Alternatively, the spot-on formulation can be prepared by encapsulation of the active ingredient to leave a residue of the therapeutic agent on the surface of the animal. These formulations will vary with regard to the weight of the therapeutic agent in the combination depending on the species of host animal to be treated, the severity and type of infection and the body weight of the host. The compounds may be administered continuously, particularly for prophylaxis, by known methods. Generally, a dose of from about 0.001 to about 10 mg per kg of body weight given as a single dose or in divided doses for a period of from 1 to 5 days will be satisfactory but, of course, there can be instance where higher or lower dosage ranges are indicated and such are within the scope of this invention. It is well within the routine skill of the practitioner to determine a particular dosing regimen for a specific host and parasite.

Preferably, a single formulation containing the compounds (A) and (B) in a substantially liquid carrier and in a form which makes possible a single application, or an application repeated a small number of times, will be administered to the animal over a highly localized region of the animal, preferably between the two shoulders. Remarkably, it has been discovered that such a formulation is highly effective against both the targeted ectoparasites and the targeted endoparasites.

The treatment is preferably carried out so as to administer to the host, on a single occasion, a dose containing between about 0.001 and about 100 mg/kg of derivative (A) and containing between about 0.1 and about 2000 µg/kg, more preferably 1000 µg/kg of compound of type (B), in particular in the case of a direct topical administration.

The amount of compound (A) for birds and animals which are small in size is preferably greater than about 0.01 mg and in a particularly preferred way between about 1 and about 50 mg/kg of weight of animal.

It also may be preferable to use controlled-release formulations. However, due to the persistence of the activity of fipronil and of compounds (B), it may be preferable for reasons of simplicity to use conventional vehicles.

This invention also provides for a method for cleaning the coats and the skin of animals by removal of the parasites which are present and of their waste and excreta. The animals treated thus exhibit a coat which is more pleasing to the eye and more pleasant to the touch.

While not wishing to be bound by theory, it is believed that the invention spot-on formulation work by the dose dissolving in the natural oils of the host's skin, fur or feathers. From there, the therapeutic agent(s) distribute around the host's body through the sebaceous glands of the skin. The therapeutic agent also remains in the sebaceous glands. Thus, the glands provide a natural reservoir for the therapeutic agent which allows for the agent to be drained back out to the follicles to reapply itself to the skin and hair. This, in turn, provides for longer time periods between application as well as not having to re-administer the dose after the host becomes wet because of rain, bathes, etc. Moreover, the inventive formulation have the further advantage in self-grooming animals of not being directly deposited of the skin or fur where the animals could orally ingest the therapeutic agent, thereby becoming sick or possibly interacting with other therapeutic agent being orally administered.

The invention also relates to such a method with a therapeutic aim intended for the treatment and prevention of parasitoses having pathogenic consequences.

In another preferred embodiment this provides for a composition for combating fleas in small mammals, in particular dogs and cats, characterized in that it contains at least one compound (A) of formula (I) as defined above and emamectin
(B), in amounts and proportions having a parasitical effectiveness for fleas and worms, in a vehicle acceptable for the animal.

The preferred class of compounds of formula (I) is that which has been defined above.

A compound of formula (I) which is very particularly preferred in the invention is 1-[2,6-Cl₂-4-CF₃ phenyl]-3-CN-4-[SO-CF₃]-5-NH₂ pyrazole.

The effective amount in a dose is, for the compound (A), preferably between about 0.001, preferentially about 0.1, and about 100 mg and in a particularly preferred way from about 1 to about 50 mg/kg of weight of animal, the higher amounts being provided for very prolonged release in or on the body of the animal.

The effective amount of compounds (B) in a dose is preferably between about 0.1 µg, preferentially about 1 µg, and about 10 mg and in a particularly preferred way from about 5 to about 200 µg/kg of weight of animal. Especially preferred is a dose between about 0.1 to about 10 mg/kg of weight of animal, with about 0.5 to 6 mg/kg being most especially preferred.

The proportions, by weight, of compound (A) and of compound (B) are preferably between about 5/1 and about 10,000/1.

The formulations of the present invention provide for the topical administration of a concentrated solution, suspension, microemulsion or emulsion for intermittent application to a spot on the animal, generally between the two shoulders (solution of spot-on type). It has been discovered that the inventive formulations are especially active against parasites when the formulations are applied to mammals and birds, especially poultry, dogs, cats, sheep, pigs, cattle and horses. These formulations comprise a composition of an effective amount of compound A and/or compound B dissolved in a pharmaceutical or veterinary acceptable carrier vehicle where a crystallization inhibitor is optionally present. Compound of (A) can advantageously be present in this formulation in a proportion of about 1 to about 20%, preferably of about 5 to about 15% (percentages as weight by volume = w/v). The liquid carrier vehicle comprises a pharmaceutically or veterinary acceptable organic solvent and optionally an organic cosolvent.

An especially preferred embodiment is spot-on formulation comprising a compound of formula (I) and emamectin or a salt thereof, with spot-on formulations comprising fipronil and emamectin being most especially preferred. It was discovered that a spot-on combination comprising a compound of formula (I) and emamectin or a salt thereof exhibited significantly greater efficacy against flea and ticks then a compound of formula (I) overtime.

Also contemplated are the pharmaceutically or veterinary acceptable acid or base salts, where applicable, of the active compounds provided for herein. The term "acid" contemplates all pharmaceutically or veterinary acceptable inorganic or organic acids. Inorganic acids include mineral acids such as hydrohalic acids, such as hydrobromic and hydrochloric acids, sulfuric acids, phosphoric acids and nitric acids. Organic acids include all pharmaceutically or veterinary acceptable aliphatic, alicyclic and aromatic carboxylic acids, dicarboxylic acids tricarboxylic acids and fatty acids. Preferred acids are straight chain or branched, saturated or unsaturated C₁-C₂₀ aliphatic carboxylic acids, which are optionally substituted by halogen or by hydroxyl groups, or C₆-C₁₂ aromatic carboxylic acids. Examples of such acids are carbonic acid, formic acid, fumaric acid, acetic acid, propionic acid, isopropionic acid, valeric acid, α-hydroxy acids, such as glycolic acid and lactic acid, chloroacetic acid, benzoic acid, methane sulfonic acid, and salicylic acid. Examples of dicarboxylic acids include oxalic acid, malic acid, succinic acid, tataric acid and maleic acid. An example of a tricarboxylic acid is citric acid. Fatty acids include all pharmaceutically or veterinary acceptable saturated or unsaturated aliphatic or aromatic carboxylic acids having 4 to 24 carbon atoms. Examples include butyric acid, isobutyric acid, sec-butyric acid, lauric acid, palmitic acid, stearic acid, oleic acid, linoleic acid, linolenic acid, and phenylsteric acid. Other acids include gluconic acid, glycoheptonic acid and lactobionic acid.

The term "base" contemplates all pharmaceutically or veterinary acceptable inorganic or organic bases. Such bases include, for example, the alkali metal and alkaline earth metal salts, such as the lithium, sodium, potassium, magnesium or calcium salts. Organic bases include the common hydrocarbyl and heterocyclic amine salts, which include, for example, the morpholine and piperidine salts.

Preferred salts for emamectin include the acid mineral salts, such as the hydrochloride, nitrate, sulfate, phosphate salts, and the organic acids such as the tartarate and malate salts. Especially preferred salts are salts of the formula: 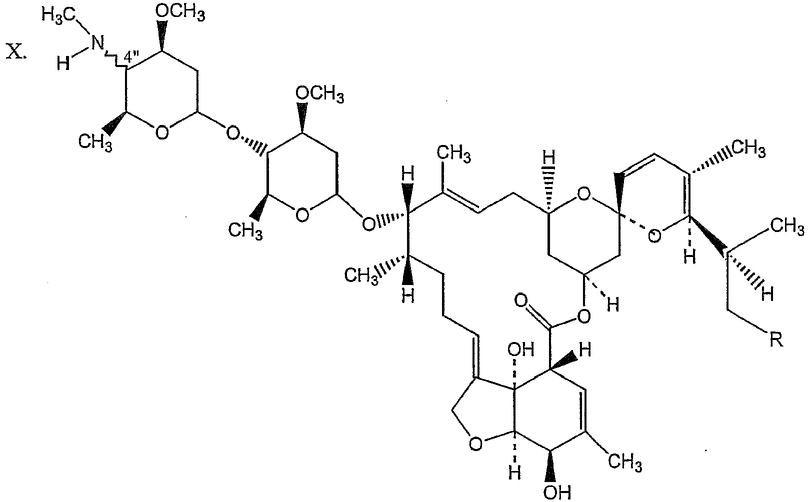 wherein
R is hydrogen or methyl; and
Xis:
   a) benzoic acid,
   b) benzoic acid substituted with one, two, or three substituents selected from the group consisting of:
      i) halogen (Cl, Br, F, I),
      ii) hydroxyl,
      iii) carboxyl,
      iv) (C₁-C₆)-alkyl, and
      v) (C₁-C₆)-alkoxyl,
   c) benzenesulfonic acid,
   d) citric acid,
   e) phosphoric acid,
   f) tartaric acid, or
   g) maleic acid.

The organic solvent for the liquid carrier vehicle will preferably have a dielectric constant of between about 10 and about 35, preferably between about 20 and about 30, the content of this solvent in the overall composition preferably representing the remainder to 100% of the composition. It is well within the skill level of the practitioner to select a suitable solvent on the basis of these parameters.

The organic cosolvent for the liquid carrier vehicle will preferably have a boiling point of less than about 100°C, preferably of less than about 80°C, and will have a dielectric constant of between about 10 and about 40, preferably between about 20 and about 30; this cosolvent can advantageously be present in the composition according to a weight/weight (w/w) ratio with respect to the solvent of between about 1/15 and about 1/2; the cosolvent is volatile in order to act in particular as drying promoter and is miscible with water and/or with the solvent. Again, it is well within the skill level of the practitioner to select a suitable solvent on the basis of these parameters.

The organic solvent for the liquid carrier includes the commonly acceptable organic solvents known in the formulation art. These solvents may be found, for example, in Remington Pharmaceutical Science, 16th Edition (1986). These solvents include, for example, acetone, ethyl acetate, methanol, ethanol, isopropanol, dimethylformamide, dichloromethane or diethylene glycol monoethyl ether (Transcutol). These solvents can be supplemented by various excipients according to the nature of the desired phases, such as C₈-C₁₀ caprylic/capric triglyceride (Estasan or Miglyol 812), oleic acid or propylene glycol.

The liquid carrier may also comprise a microemulsion. Microemulsions are also well suited as the liquid carrier vehicle. Microemulsions are quaternary systems comprising an aqueous phase, an oily phase, a surfactant and a cosurfactant. They are translucent and isotropic liquids.

Microemulsions are composed of stable dispersions of microdroplets of the aqueous phase in the oily phase or conversely of microdroplets of the oily phase in the aqueous phase. The size of these microdroplets is less than 200 nm (1000 to 100,000 nm for emulsions). The interfacial film is composed of an alternation of surface-active (SA) and co-surface-active (Co-SA) molecules which, by lowering the interfacial tension, allows the microemulsion to be formed spontaneously.

The oily phase can in particular be formed from mineral or vegetable oils, from unsaturated polyglycosylated glycerides or from triglycerides, or alternatively from mixtures of such compounds. The oily phase preferably comprises triglycerides and more preferably medium-chain triglycerides, for example C₈-C₁₀ caprylic/capric triglyceride. The oily phase will represent, in particular, from about 2 to about 15%, more particularly from about 7 to about 10%, preferably from about 8 to about 9%, v/v of the microemulsion.

The aqueous phase includes, for example water or glycol derivatives, such as propylene glycol, glycol ethers, polyethylene glycols or glycerol. Propylene glycol, diethylene glycol monoethyl ether and dipropylene glycol monoethyl ether are especially preferred. Generally, the aqueous phase will represent a proportion from about 1 to about 4% v/v in the microemulsion.

Surfactants for the microemulsion include diethylene glycol monoethyl ether, dipropyelene glycol monomethyl ether, polyglycolysed C₈-C₁₀ glycerides or polyglyceryl-6 dioleate. In addition to these surfactants, the cosurfactants include short-chain alcohols, such as ethanol and propanol.

Some compounds are common to the three components discussed above, i.e., aqueous phase, surfactant and cosurfactant. However, it is well within the skill level of the practitioner to use different compounds for each component of the same formulation.

The cosurfactant to surfactant ratio will preferably be from about 1/7 to about 1/2. There will preferably be from about 25 to about 75% v/v of surfactant and from about 10 to about 55% v/v of cosurfactant in the microemulsion.

Likewise, the co-solvents are also well known to a practitioner in the formulation art. Preferred co-solvents are those which is a promoter of drying and include, for example, absolute ethanol, isopropanol (2-propanol) or methanol.

The crystallization inhibitor can in particular be present in a proportion of about 1 to about 20% (w/v), preferably of about 5 to about 15%. The inhibitor preferably corresponds to the test in which 0.3 ml of a solution comprising 10% (w/v) of the compound of formula (I) in the liquid carrier and 10% of the inhibitor are deposited on a glass slide at 20°C and allowed to stand for 24 hours. The slide is then observed with the naked eye. Acceptable inhibitors are those whose addition provides for few or no crystals, and in particular less than 10 crystals, preferably 0 crystals.

Although this is not preferred, the formulation can optionally comprise water, in particular in a proportion of 0 to about 30% (volume by volume v/v), in particular of 0 to about 5%.

The formulation can also comprise an antioxidizing agent intended to inhibit oxidation in air, this agent being in particular present in a proportion of about 0.005 to about 1% (w/v), preferably of about 0.01 to about 0.05%.

Crystallization inhibitors which can be used in the invention include:
- polyvinylpyrrolidone, polyvinyl alcohols, copolymers of vinyl acetate and of vinylpyrrolidone, polyethylene glycols, benzyl alcohol, mannitol, glycerol, sorbitol or polyoxyethylenated esters of sorbitan; lecithin or sodium carboxymethylcellulose; or acrylic derivatives, such as methacrylates and others,
- anionic surfactants, such as alkaline stearates, in particular sodium, potassium or ammonium stearate; calcium stearate or triethanolamine stearate; sodium abietate; alkyl sulphates, in particular sodium lauryl sulphate and sodium cetyl sulphate; sodium dodecylbenzenesulphonate or sodium dioctyl sulphosuccinate; or fatty acids, in particular those derived from coconut oil,
- cationic surfactants, such as water-soluble quaternary ammonium salts of formula N⁺R'R"R"'R""Y⁻, in which the R radicals are identical or different optionally hydroxylated hydrocarbon radicals and Y⁻ is an anion of a strong acid, such as halide, sulphate and sulphonate anions; cetyltrimethylammonium bromide is one of the cationic surfactants which can be used,
- amine salts of formula N⁺R'R"R"', in which the R radicals are identical or different optionally hydroxylated hydrocarbon radicals; octadecylamine hydrochloride is one of the cationic surfactants which can be used,
- non-ionic surfactants, such as optionally polyoxyethylenated esters of sorbitan, in particular Polysorbate 80, or polyoxyethylenated alkyl ethers; polyethylene glycol stearate, polyoxyethylenated derivatives of castor oil, polyglycerol esters, polyoxyethylenated fatty alcohols, polyoxyethylenated fatty acids or copolymers of ethylene oxide and of propylene oxide,
- amphoteric surfactants, such as substituted lauryl compounds of betaine,
- or preferably a mixture of at least two of the compounds listed above.

In a particularly preferred embodiment, a crystallization inhibitor pair will be used. Such pairs include, for example, the combination of a film-forming agent of polymeric type and of a surface-active agent. These agents will be selected in particular from the compounds mentioned above as crystallization inhibitor.

Particularly preferred film-forming agents of polymeric type include:
- the various grades of polyvinylpyrrolidone,
- polyvinyl alcohols, and
- copolymers of vinyl acetate and of vinylpyrrolidone.

Especially preferred surface-active agents, include those made of non-ionic surfactants, preferably polyoxyethylenated esters of sorbitan and in particular the various grades of polysorbate, for example Polysorbate 80.

The film-forming agent and the surface-active agent can in particular be incorporated in similar or identical amounts within the limit of the total amounts of crystallization inhibitor mentioned elsewhere.

The pair thus constituted secures, in a noteworthy way, the objectives of absence of crystallization on the coat and of maintenance of the cosmetic appearance of the fur, that is to say without a tendency towards sticking or towards a sticky appearance, despite the high concentration of active material.

Particularly preferred antioxidizing agents are those conventional in the art and include, for example, butylated hydroxyanisole, butylated hydroxytoluene, ascorbic acid, sodium metabisulphite, propyl gallate, sodium thiosulphate or a mixture of not more than two of them.

The formulation adjuvants discussed above are well known to the practitioner in this art and may be obtained commercially or through known techniques. These concentrated compositions are generally prepared by simple mixing of the constituents as defined above; advantageously, the starting point is to mix the active material in the main solvent and then the other ingredients or adjuvants are added.

The volume applied can be of the order of about 0.3 to about 1 ml, preferably of the order of about 0.5 ml, for cats and of the order of about 0.3 to about 3 ml for dogs, depending on the weight of the animal.

The formulations according to the invention are extremely effective for long durations of time in the treatment of parasites such as fleas of mammals and, in particular, of small mammals such as dogs and cats. The inventive formulations exhibit a degree of effectiveness against other parasitic insects and in particular ticks, mites, mosquitoes and flies. Moreover, the inventive formulations are also extremely effective for a long duration in the treatment of endoparasites, such as the dirofilariasis parasite and/or heartworms and/or roundworms. The inventive formulations further exhibit synergy when treating infestations cause by ectoparasites and endoparasites. Also disclosed is a synergistic formulation for the treatment of filariae and heartworms and roundworms comprises fipronil and milbemectin, fipronil and selamectin, fipronil and emamectin, fipronil and latidectin or fipronil and lepimectin.

This invention also provides for the use of at least one compound of formula (I) and of at least one compound of type (B), as defined above, in the preparation of a composition as defined above.

### EXAMPLES

### EXAMPLE 1: Preparation of a concentrated solution for intermittent application (spot-on) (not according to the invention)

A concentrated solution for cutaneous application is prepared which contains, as weight by volume of solution, 10% of fipronil and 0.25% of ivermectin. The administration volume is 1 ml per 10 kg of animal weight. The composition is as follows, as weight/volume:
- fipronil: 10%
- ivermectin 0.25%
- Polyvinylpyrrolidone (Kollidon 17 PF): 5%
- Polysorbate 80 (Tween 80): 5%
- ethanol: 10%
- Transcutol: q.s. for 100%

### EXAMPLE 1a: Preparation of a concentrated solution for intermittent application (spot-on) (not according to the invention)

A concentrated solution for cutaneous application may be prepared which contains, as weight by volume of solution, 10% of fipronil and 0.25% of latidectin. The administration volume can be 1 ml per 10 kg of animal weight. The composition may be as follows, as weight/volume:
- fipronil: 10%
- latidectin 0.25%
- Polyvinylpyrrolidone (Kollidon 17 PF): 5%
- Polysorbate 80 (Tween 80): 5%
- ethanol: 10%
- Transcutol: q.s. for 100%

### EXAMPLE 1b: Preparation of a concentrated solution for intermittent application (spot-on) (not according to the invention)

A concentrated solution for cutaneous application may be prepared which contains, as weight by volume of solution, 10% of fipronil and 0.25% of lepimectin. The administration volume can be 1 ml per 10 kg of animal weight. The composition may be as follows, as weight/volume:
- fipronil: 10%
- lepimectin 0.25%
- Polyvinylpyrrolidone (Kollidon 17 PF): 5%
- Polysorbate 80 (Tween 80): 5%
- ethanol: 10%
- Transcutol: q.s. for 100%

### EXAMPLE 2: Preparation of a concentrated microemulsion for intermittent application (spot-on) (not according to the invention)

The ingredients used are as follows:
- oily phase: C₈-C₁₀ caprylic/capric triglyceride (Estasan)
- aqueous phase: propylene glycol
- surfactant: diethylene glycol monoethyl ether (Transcutol)
- cosurfactant: ethanol or 2-propanol
- crystallization inhibitor pair: Polysorbate 80 (Tween 80) and polyvinylpyrrolidone (Kollidon 17 PF).

A composition example contains:
- fipronil: 10 g
- ivermectin: 0.5 g
- Estasan: 8.5 ml
- Transcutol: 60 ml
- ethanol: 15 ml
- Kollidon 17 PF: 5 g
- Tween 80: 5 g
- propylene glycol: q.s. for 100 ml.

In the formulation described, the Transcutol acts as the surfactant (SA) and the ethanol or 2-propanol acts as cosurfactant (Co-SA). They make it possible to obtain, from a mixture of medium-chain triglycerides (Estasan) which is immiscible with propylene glycol, an isotropic transparent microemulsion. The crystallization inhibitor pair will be added once the microemulsion has been formed.

### EXAMPLE 2a: Preparation of a concentrated microemulsion for intermittent application (spot-on) (not according to the invention)

The ingredients which may be used are as follows:
- oily phase: C₈-C₁₀ caprylic/capric triglyceride (Estasan)
- aqueous phase: propylene glycol
- surfactant: diethylene glycol monoethyl ether (Transcutol)
- cosurfactant: ethanol or 2-propanol
- crystallization inhibitor pair: Polysorbate 80 (Tween 80) and polyvinylpyrrolidone (Kollidon 17 PF).

A composition example may contain:
- fipronil: 10 g
- latidectin: 0.5 g
- Estasan: 8.5 ml
- Transcutol: 60 ml
- ethanol: 15 ml
- Kollidon 17 PF: 5 g
- Tween 80: 5 g
- propylene glycol: q.s. for 100 ml.

In the formulation described, the Transcutol acts as the surfactant (SA) and the ethanol or 2-propanol acts as cosurfactant (Co-SA). They make it possible to obtain, from a mixture of medium-chain triglycerides (Estasan) which is immiscible with propylene glycol, an isotropic transparent microemulsion. The crystallization inhibitor pair will be added once the microemulsion has been formed.

### EXAMPLE 2b: Preparation of a concentrated microemulsion for intermittent application (spot-on) (not according to the invention)

The ingredients which may be used are as follows:
- oily phase: C₈-C₁₀ caprylic/capric triglyceride (Estasan)
- aqueous phase: propylene glycol
- surfactant: diethylene glycol monoethyl ether (Transcutol)
- cosurfactant: ethanol or 2-propanol
- crystallization inhibitor pair: Polysorbate 80 (Tween 80) and polyvinylpyrrolidone (Kollidon 17 PF).

A composition example may contain:
- fipronil: 10 g
- lepimectin: 0.5 g
- Estasan: 8.5 ml
- Transcutol: 60 ml
- ethanol: 15 ml
- Kollidon 17 PF: 5 g
- Tween 80: 5 g
- propylene glycol: q.s. for 100 ml.

In the formulation described, the Transcutol acts as the surfactant (SA) and the ethanol or 2-propanol acts as cosurfactant (Co-SA). They make it possible to obtain, from a mixture of medium-chain triglycerides (Estasan) which is immiscible with propylene glycol, an isotropic transparent microemulsion. The crystallization inhibitor pair will be added once the microemulsion has been formed.

### EXAMPLE 3: (not according to the invention)

Five dogs weighing 12 kg, receive the application of 1 ml of composition according to Example 2 or 3, i.e. 100 mg of fipronil and 2.5 mg of ivermectin, by localized cutaneous application between the two shoulders. The measurements carried out on the plasma of the animals show the production of an ivermectin peak of 1000 to 1500 to 2000 pg/ml.

A monthly or even bimonthly treatment of dogs makes possible complete control of fleas, ticks and dirofilariasis parasites.

### EXAMPLE 4: Plasma kinetics of ivermectin after oral administration of Cardomec® at the dose of 6 µg.kg⁻¹ to dogs (not according to the invention)

This example was conducted in order to demonstrate the pharmacokinetics of ivermectin in the dog after oral administration of Cardomec^{®} at a dose of 6 *µ*g.kg⁻¹ of ivermectin which is known to be 100% effective on heartworms and to have a route of reference for further development.

Five male Beagle dogs received a dose of approximately 6 *µ*g.kg⁻¹ of ivermectin, i.e., one 68 *µ*g Cardomec® tablet per dog. The animals were fasted prior to administration and up to 6 hours after treatment to prevent a possible interaction with food.

Blood samples were collected at intervals up to 28 days post-dosing. The determination of ivermectin in dog plasma was carried out by HPLC using fluorescence detection after derivatization. The limit of quantification was 100 pg.ml⁻¹.

Ivermectin could be quantified in dog plasma up to day 1 or 7, depending on the dog (Table 1). The profiles were very variable, presenting a first order absorption with one or two peaks and a biexponential depletion.

The pharmacokinetic parameters were very variable (Tables 2 and 3). Cₘₐₓ ranged between 422 and 2964 pg.ml⁻¹, tₘₐₓ between 3 and 12 h, AUC between 9164 and 90938 pg.h.ml⁻¹. The ratio between the highest and smallest Cₘₐₓ was 7, the differences even more striking for AUC(₀₋ₜ) and AUC with ratios of 17 and 10 respectively. Compared with the other parameters, the terminal half-lives (t1/2) were relatively similar between animals (CV < 40 %), ranging between 26.0 and 64.5 h, i.e. 1.08 day and 2.69 days. The mean value of the terminal half-life was 40.1 h (1.67 d).

The mean pharmacokinetic parameters determined from the present study are in good agreement with the literature: 3 h for tₘₐₓ compared with the literature data of 2-4 hours, t1/2 = 1.67 d against 1.6 to 1.8 d in the literature. Cₘₐₓ and AUC only were low with values of 1362 pg.ml⁻¹ and 44604 pg.h.ml⁻¹ *versus* mean literature values of more than 2000 pg. ml⁻¹ and 107318 pg.h.ml⁻¹⁻, respectively. This experiment was conducted on fasted animals. Some prior articles mentioned a possible interaction of ivermectin with food, without the food state of the dogs being specified. It is possible that they were unfasted and that food interacts positively with ivermectin absorption, i.e., increases the rate and extent of absorption of ivermectin and therefore leads to higher Cₘₐₓ and AUC than in the present experiment.

Two conclusions can be drawn from this study: the inter-animal variability is an important feature and the terminal half-lives are relatively constant (around 2 days).

### EXAMPLE 5: Efficacy against ticks of a topical composition according to the present invention against ticks.

The following composition according to the present invention was prepared:

| Inventive Composition | |
|---|---|
| Component | % Component (w/v) |
| Fipronil | 10% |
| Emamectin | 0.5% |
| Ethanol | 10% |
| Polyvidene | 5% |
| Tween 80 | 5% |
| BHT | 0.01% |
| BHA | 0.02% |
| Diethylene Glycol | qs 100% |
| Monomethyl ether | |

The following comparison topical composition was prepared:

**COMPARATIVE - EXAMPLE 1:**

| PLACEBO | |
|---|---|
| Component | % Component (w/v) |
| Ethanol | 10% |
| Polyvidone | 5% |
| Tween 80 | 5% |
| BHT | 0.01% |
| BHA | 0.02% |
| Diethylene Glycol | qs 100% |
| Monomethyl ether | |

**COMPARATIVE - EXAMPLE 2**

| FRONTLINE | |
|---|---|
| Component | % Component (w/v) |
| Fipronil | 10% |
| Ethanol | 10% |
| Polyvidone | 5% |
| Tween 80 | 5% |
| BHT | 0.01% |
| BHA | 0.02% |
| Diethylene Glycol | qs 100% |
| Monomethyl ether | |

**COMPARATIVE - EXAMPLE 3**

| EMAMECTIN | |
|---|---|
| Component | % Component (w/v) |
| Emamectin | 0.5% |
| Ethanol | 10% |
| Polyvidone | 5% |
| Tween 80 | 5% |
| BHT | 0.01% |
| BHA | 0.02% |
| Diethylene Glycol | qs 100% |
| Monomethyl ether | |

The inventive composition and the comparative composition were tested for their effectiveness on the flea and tick infestations on dogs. Beagles were infested with approximately 50 *Rhipicephalus sanguineus* (ticks) on days 14, 20, 27, 34, 41, 48 and 62 and with approximately 100 *Ctenocephalides felis* on days 15, 28, 42, and 70. The fleas were counted and removed 24 hour after each infestation and the ticks were counted and removed from all dogs after 48 hours of infestation. There were four dogs in each group. A topical formulation dosage rates 1.0 ml/10 kg of body weight of the topical formulation was applied on the midline of the neck, between the base of the skull and the shoulder blade. Table I reports the average percent efficiency against flea and tick counts for each of the biological example compared to the placebo.

**Table 1**

| Composition | Fleas Day 16 | Ticks Day 16 | Ticks Day 22 | Fleas Day 29 | Ticks Day 36 | Ticks Day 36 | Fleas Day 43 | Ticks Day 43 | Ticks Day 50 | Ticks Day 64 | Fleas Day 71 |
|---|---|---|---|---|---|---|---|---|---|---|---|
| Comparative 2 | 100.0 | 100.0 | 100.0 | 100.0 | 93.6 | 99.2 | 96.4 | 85.8 | 74.5 | 54.9 | 32.9 |
| Comparative 3 | 31.2 | 29.4 | 35.0 | ND | ND | ND | ND | ND | 38.8 | 36.9 | 0.3 |
| Inventive Composition | 100.0 | 99.4 | 100.0 | 100.0 | 98.2 | 100.0 | 98.9 | 95.3 | 88.9 | 64.8 | 72.2 |

The data presented in Table I demonstrate that the inventive composition exhibits a far greater efficacy against fleas and ticks than either fipronil or emamectin alone. Fig. 1 depicts the comparison of the inventive composition against a composition comprising fipronil against ticks. Fig. 2 depicts the comparison of the inventive composition against a composition comprising fipronil against fleas:

## Claims

1. A spot-on formulation for the treatment or prophylaxis of parasite infestation in mammals or birds which comprises
(1) a composition comprising
(A) an effective amount of (1) 1-[2,6-Cl₂-4-CF₃ phenyl]-3-CN-4-[SO-CF₃]-5-NH₂ pyrazole (fipronil); and
(B) an effective amount of emamectin or a salt thereof;
(2) a pharmaceutically or veterinary acceptable liquid carrier vehicle wherein the liquid carrier vehicle comprises a solvent and a cosolvent wherein the solvent is selected from the group consisting of acetone, acetonitrile, benzyl alcohol, butyl diglycol, dimethylacetamide, dimethylformamide, dipropylene glycol n-butyl ether, ethanol, isopropanol, methanol, ethylene glycol monoethyl ether, ethylene glycol monomethyl ether, monomethylaceamide, dipropylene glycol monomethyl ether, liquid polyoxyethylene glycols, propylene glycol, 2-pyrrolidone, diethylene glycol monoethyl ether, ethylene glycol, diethyl phthalate, and a mixture of at least two of these solvents and the cosolvent is selected from the group consisting of absolute ethanol, isopropanol or methanol;
(3) a crystallization inhibitor, wherein the crystallization inhibitor is selected from the group consisting of an anionic surfactant, a cationic surfactant, a non-ionic surfactant, an amine salt, an amphoteric surfactant, polyvinylpyrrolidone, polyvinyl alcohols, copolymers of vinyl acetate and vinylpyrrolidone, polyethylene glycols, benzyl alcohol, mannitol, glycerol, sorbitol, polyoxyethylenated sorbitan esters; lecithin, sodium carboxymethylcellulose, and acrylic derivatives, or a mixture of these crystallization inhibitors.

2. The spot-on formulation according to claim 1, wherein the liquid carrier vehicle comprises a microemulsion.

3. The spot-on formulation according to claim 1, wherein the liquid carrier vehicle further comprises a diluent.

4. The spot-on formulation according to claim 1, wherein the combination comprises 0.001 to 100 mg/kg of weight of mammal or bird of (1) 1-[2,6-Cl₂-4-CF₃ phenyl]-3-CN-4-[SO-CF₃]-5-NH₂ pyrazole and between 0.1 mg to 10 mg/kg of weight of mammal or bird of emamectin or a salt thereof.

5. The spot-on formulation according to claim 1, wherein the combination comprises 1 to 50 mg/kg of weight of mammal or bird of (1) 1-[2,6-Cl₂-4-CF₃ phenyl]-3-CN-4-[SO-CF₃]-5-NH₂ pyrazole and between 0.1 µg to 1 mg/kg per weight of mammal or bird of emamectin or a salt thereof.

6. The spot-on formulation according to claim 1, wherein the combination comprises between 5 and 500 µg/kg per weight of mammal or bird of emamectin or a salt thereof.

7. The spot-on formulation according to claim 4 which comprises 0.5 mg/kg of emamectin per weight of mammal or bird.

8. The spot-on formulation according to claim 1, wherein the combination comprises the ratio, by weight, of (1) 1-[2,6-Cl₂-4-CF₃ phenyl]-3-CN-4-[SO-CF₃]-5-NH₂ pyrazole to emamectin or a salt thereof is 5/1 to 10,000/1.

9. The spot-on formulation according to claim 1, which further comprises an antioxidant wherein 0.005 to 1% (w/v) of antioxidant is present and the antioxidant is selected from the group consisting of butylated hydroxyanisole, butylated hydroxytoluene, ascorbic acid, sodium metabisulphite, propyl gallate, and sodium thiosulphate.

10. The spot-on formulation according to claim 1, which further comprises water wherein water is present in a proportion of from 0 to 30% (v/v).

11. The spot-on formulation according to claim 1, wherein the crystallization inhibitor is present in an amount from 1 to 20% (w/v) and wherein the crystallization inhibitor is a crystallization inhibitor system comprising a polymeric film-forming agent and a surfactant, wherein the polymeric film-forming agent is polyvinylpyrrolidone, polyvinyl alcohols, or a copolymer of vinyl acetate and polyvinylpyrrolidone and the surfactant is a non- ionic surfactant.

12. The spot-on formulation according to claim 1, wherein
- the anionic surfactant is alkaline stearates, sodium abietate; alkyl sulphates; sodium dodecylbenzenesulphonate, sodium dioctylsulphosuccinate; and fatty acids;
- the cationic surfactant is water-soluble quaternary ammonium salts of formula N⁺R'R"R"'R""Y⁻ in which the radicals R independently are hydrocarbon radicals, optionally hydroxylated, and Y⁻ is an anion of a strong acid;
- the amine salt is an amine salt of N⁺R'R"R"' in which the radicals R independently are optionally hydroxylated hydrocarbon radicals;
- the non-ionic surfactant is optionally polyoxyethylenated sorbitan esters, polyoxyethylenated alkyl ethers; polyethylene glycol stearate, polyoxyethylenated derivatives of castor oil, polyglycerol esters, polyoxyethylenated fatty alcohols, polyoxyethylenated fatty acids, copolymers of ethylene oxide and propylene oxide; and
- the amphoteric surfactant is lauryl-substituted betaine compounds.

13. A spot-on composition, which comprises, in a veterinarily acceptable vehicle, an amount parasitically effective of at least one compound (A), and an amount parasitically effective of at least one compound (B), wherein:
compound (A) is of the formula (I) 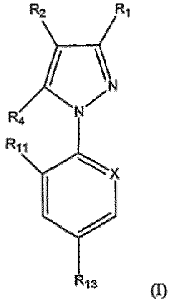
in which:
R₁ is CN;
R₂ is S(O)ₙR₃;
R₃ is haloalkyl;
R₄ represents NH₂;
R₁₁ represents halogen atom;
R₁₃ represents haloalkyl;
n represents an integer equal to 0, 1 or 2;
X represents a radical C-R₁₂;
R₁₂ represents a halogen atom;
and
compound (B) is an endectocidal parasiticide which is emamectin, or a salt thereof; and,
the vehicle is for a localized cutaneous application to the animal between the shoulders and contains an organic solvent, an organic cosolvent and/or a crystallization inhibitor wherein:
the crystallization inhibitor is selected from the group consisting of polyvinylpyrrolidone, copolymers of vinyl acetate and vinylpyrrolidone, polyoxyethylenated sorbitan esters and mixtures thereof;
the organic solvent comprises acetone, ethyl acetate, methanol, ethanol, isopropanol, dimethylformamide, dicholoromethane or diethyl glycol monoethyl ether; said solvent optionally supplemented by C₈-C₁₀ caprylic/capric triglyceride, oleic acid or propylene glycol; and
the organic cosolvent selected from the group consisting of ethanol, isopropanol, and methanol;
for the use in combating parasites of a cat or dog comprising localized cutaneous application to the cat or dog, between the shoulders, at a frequency not greater than monthly, whereby there is a prolonged relase of compound (A) in or on the body of the cat or dog and there is a measurable plasma level of compound (B) in the cat or dog.

14. The spot-on composition for use of claim 13 wherein compound (A) is 1-[4-CF₃ 2,6-Cl₂ phenyl] 3-cyano 4-[CF₃-SO] 5-NH₂ pyrazole.

15. The spot-on composition for use of claim 14 wherein compound (A) is present in an amount of from 0.1 to 100 mg/kg of weight of animal and compound (B) is present in the spot-on composition in an amount of from 1 µg to 1 mg/kg of weight of animal.

16. The spot-on composition for use of claim 13 wherein the crystallization inhibitor is a crystallization inhibitor pair.

17. The spot-on composition according to claim 13 wherein the organic solvent is diethylene glycol monoethyl ether and the organic cosolvent is ethanol or isopropanol.

18. The spot-on composition for use of claim 14 wherein there is a weight/weight ratio of the cosolvent/solvent and the weight/weight ratio of the cosolvent/solvent is between 1/15 and 1/2.

19. The spot-on composition for use of claim 14 further comprising water in an amount of less than 30% (v/v).

20. The spot-on composition for use of claim 14 wherein the spot-on composition does not contain water.

21. The spot-on composition for use of claim 14 further comprising an antioxidant.

22. The spot-on composition for use of claim 21 wherein the antioxidant is present in an amount of 0.005 to 1 % (w/v) and is selected from the group consisting of butylated hydroxyanisole, butylated hydroxytoluene, ascorbic acid, sodium metabisulphite, propyl gallate, and sodium thiosulphate.

23. Use of a spot-on composition, which comprises, an amount parasitically effective of at least one compound (A), and an amount parasitically effective of at least one compound (B), wherein:
compound (A) is of the formula (I) 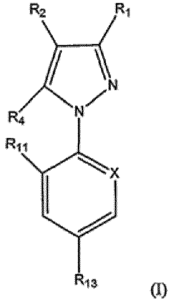
in which:
R₁ is CN;
R₂ is S(O)ₙR₃;
R₃ is haloalkyl;
R₄ represents NH₂;
R₁₁ represents halogen atom;
R₁₃ represents haloalkyl;
n represents an integer equal to 0, 1 or 2;
X represents a radical C-R₁₂;
R₁₂ represents a halogen atom;
and
compound (B) is an endectocidal parasiticide which is emamectin, or a salt thereof,
for the manufacture of a medicament which comprises a veterinarily acceptable vehicle for combating parasites of a cat or dog comprising localized cutaneous application to the cat or dog, between the shoulders, at a frequency not greater than monthly, wherein the vehicle is for a localized cutaneous application to the animal between the shoulders and contains an organic solvent, an organic cosolvent and/or a crystallisation inhibitor wherein:
the crystallisation inhibitor selected from the group consisting of polyvinylpyrrolidone, copolymers of vinyl acetate and vinylpyrrolidone, polyoxyethylenated sorbitan esters and mixtures thereof;
the organic solvent comprises acetone, ethyl acetate, methanol, ethanol, isopropanol, dimethylformamide, dichloromethane or diethyl glycol monoethyl ether; said solvent optionally supplemented by C₈-C₁₀ caprylic/capric triglyceride, oleic acid or propylene glycol; and
the organic cosolvent is selected from the group consisting of ethanol, isopropanol, and methanol;
whereby there is a prolonged release of compound (A) in or on the body of the cat or dog and there is a measurable plasma level of compound (B) in the cat or dog.

24. The use according to claim 23, wherein compound (A) is 1-[4-CF₃ 2,6-Cl₂ phenyl] 3-cyano 4-[CF₃-SO] 5-NH₂ pyrazole.

## Patentansprüche

1. Eine Spot-on-Formulierung zur Behandlung oder Prophylaxe von Parasitenbefall bei Säugetieren oder Vögeln, die
(1) eine Zusammensetzung, umfassend
(A) eine wirksame Menge von (1) 1-[2,6-Cl₂-4-CF₃-phenyl]-3-CN-4-[SO-CF₃]-5-NH₂-pyrazol (Fipronil); und
(B) eine wirksame Menge an Emamectin oder einem Salz davon;
(2) ein pharmazeutisch oder veterinärmedizinisch verträgliches flüssiges Trägervehikel, wobei das flüssige Trägervehikel ein Lösungsmittel und ein Colösungsmittel umfasst, wobei das Lösungsmittel ausgewählt ist aus der Gruppe, bestehend aus Aceton, Acetonitril, Benzylalkohol, Butyldiglykol, Dimethylacetamid, Dimethylformamid, Dipropylenglykol-n-butylether, Ethanol, Isopropanol, Methanol, Ethylenglykolmonoethylether, Ethylenglykolmonomethylether, Monomethylacetamid, Dipropylenglykolmonomethylether, flüssige Polyoxyethylenglykole, Propylenglykol, 2-Pyrrolidon, Diethylenglykolmonoethylether, Ethylenglykol, Diethylphthalat und einem Gemisch aus mindestens zwei dieser Lösungsmittel, und das Colösungsmittel ausgewählt ist aus der Gruppe, bestehend aus absolutem Ethanol, Isopropanol oder Methanol;
(3) einen Kristallisationsinhibitor, wobei der Kristallisationsinhibitor ausgewählt ist aus der Gruppe, bestehend aus einem anionischen grenzflächenaktiven Mittel, einem kationischen grenzflächenaktiven Mittel, einem nichtionischen grenzflächenaktiven Mittel, einem Aminsalz, einem amphoteren grenzflächenaktiven Mittel, Polyvinylpyrrolidon, Polyvinylalkoholen, Copolymeren von Vinylacetat und Vinylpyrrolidon, Polyethylenglykole, Benzylalkohol, Mannit, Glycerin, Sorbit, polyoxyethylenierte Sorbitanester; Lecithin, Natriumcarboxymethylcellulose und Acrylderivate oder einem Gemisch dieser Kristallisationsinhibitoren; umfasst.

2. Die Spot-on-Formulierung gemäß Anspruch 1, wobei das flüssige Trägervehikel eine Mikroemulsion umfasst.

3. Die Spot-on-Formulierung gemäß Anspruch 1, wobei das flüssige Trägervehikel ferner ein Verdünnungsmittel umfasst.

4. Die Spot-on-Formulierung gemäß Anspruch 1, wobei die Kombination 0,001 bis 100 mg/kg des Gewichts des Säugetiers oder Vogels von (1) 1-[2,6-Cl₂-4-CF₃-phenyl]-3-CN-4-[SO-CF₃]-5-NH₂-pyrazol und zwischen 0,1 mg bis 10 mg/kg des Gewichts des Säugetieres oder des Vogels von Emamectin oder einem Salz davon umfasst.

5. Die Spot-on-Formulierung gemäß Anspruch 1, wobei die Kombination 1 bis 50 mg/kg des Gewichts des Säugetiers oder Vogels von (1) 1-[2,6-Cl₂-4-CF₃-phenyl]-3-CN-4-[SO-CF₃]-5-NH₂-pyrazol und zwischen 0,1 µg bis 1 mg/kg des Gewichts des Säugetieres oder Vogels von Emamectin oder einem Salz davon umfasst.

6. Die Spot-on-Formulierung gemäß Anspruch 1, wobei die Kombination zwischen 5 und 500 µg/kg des Gewichts des Säugetiers oder Vogels von Emamectin oder einem Salz davon umfasst.

7. Die Spot-on-Formulierung gemäß Anspruch 4, die 0,5 mg/kg Emamectin pro Gewicht des Säugetiers oder Vogels enthält.

8. Die Spot-on-Formulierung gemäß Anspruch 1, wobei die Kombination das Gewichtsverhältnis von (1) 1-[2,6-Cl₂-4-CF₃-phenyl]-3-CN-4-[SO-CF₃]-5-NH₂-pyrazol zu Emamectin oder einem Salz davon umfasst, das 5/1 bis 10.000/1 beträgt.

9. Die Spot-on-Formulierung gemäß Anspruch 1, die ferner ein Antioxidans enthält, wobei 0,005 bis 1% (Gew./Vol.) Antioxidans vorliegt und das Antioxidans ausgewählt ist aus der Gruppe bestehend aus butyliertem Hydroxyanisol, butyliertem Hydroxytoluol, Ascorbinsäure, Natriummetabisulfit, Propylgallat und Natriumthiosulfat.

10. Die Spot-on-Formulierung gemäß Anspruch 1, die ferner Wasser umfasst, wobei Wasser in einem Anteil von 0 bis 30% (Vol./Vol.) vorhanden ist.

11. Die Spot-on-Formulierung gemäß Anspruch 1, wobei der Kristallisationsinhibitor in einer Menge von 1 bis 20% (Gew./Vol.) vorliegt und wobei der Kristallisationsinhibitor ein Kristallisationsinhibitorsystem, umfassend ein polymeres filmbildendes Mittel und ein grenzflächenaktives Mittel, ist, wobei das polymere filmbildende Mittel Polyvinylpyrrolidon, Polyvinylalkohole oder ein Copolymer aus Vinylacetat und Polyvinylpyrrolidon ist und das grenzflächenaktive Mittel ein nichtionisches grenzflächenaktives Mittel ist.

12. Die Spot-on-Formulierung gemäß Anspruch 1, wobei
- das anionische grenzflächenaktive Mittel alkalische Stearate, Natriumabietat, Alkylsulfate, Natriumdodecylbenzolsulfonat, Natriumdioctylsulfosuccinat, und Fettsäuren ist;
- das kationische grenzflächenaktive Mittel wasserlösliche quaternäre Ammoniumsalze der Formel N⁺R'R"R"'R""Y⁻ ist, in der die Reste R unabhängig voneinander Kohlenwasserstoffreste sind, gegebenenfalls hydroxyliert, und Y⁻ ein Anion einer starken Säure ist;
- das Aminsalz ein Aminsalz von N⁺R'R"R"' ist, in dem die Reste R unabhängig voneinander gegebenenfalls hydroxylierte Kohlenwasserstoffreste sind;
- das nichtionische grenzflächenaktive Mittel gegebenenfalls polyoxyethylenierte Sorbitanester, polyoxyethylenierte Alkylether, Polyethylenglykolstearat, polyoxyethylenierte Derivate von Rizinusöl, Polyglycerinester, polyoxyethylenierte Fettalkohole, polyoxyethylenierte Fettsäuren, Copolymere von Ethylenoxid und Propylenoxid ist; und
- das amphotere grenzflächenaktive Mittel laurylsubstituierte Betainverbindungen ist.

13. Eine Spot-on-Zusammensetzung, die in einem veterinärmedizinisch verträglichen Träger, eine parasitär wirksame Menge von mindestens einer Verbindung (A) und eine parasitär wirksame Menge von mindestens einer Verbindung (B) umfasst, wobei:
Verbindung (A) der Formel (I) entspricht 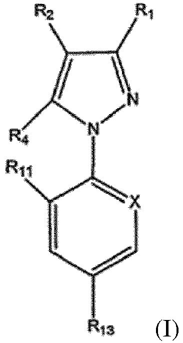
in der:
R₁ CN ist;
R₂ S(O)ₙR₃ ist;
R₃ Halogenalkyl ist;
R₄ NH₂ darstellt;
R₁₁ Halogenatom darstellt;
R₁₃ Halogenalkyl darstellt;
n eine ganze Zahl gleich 0, 1 oder 2 darstellt;
X einen Rest C-R₁₂ darstellt;
R₁₂ ein Halogenatom darstellt;
und
Verbindung (B) ein endektizides Parasitizid ist, das Emamectin oder ein Salz davon ist; und
der Träger für eine lokalisierte kutane Applikation auf das Tier zwischen den Schultern ist und ein organisches Lösungsmittel, ein organisches Colösungsmittel und/oder einen Kristallisationsinhibitor enthält, wobei:
der Kristallisationsinhibitor ausgewählt ist aus der Gruppe bestehend aus Polyvinylpyrrolidon, Copolymeren von Vinylacetat und Vinylpyrrolidon, polyoxyethylenierten Sorbitanestern und Mischungen davon;
das organische Lösungsmittel Aceton, Ethylacetat, Methanol, Ethanol, Isopropanol, Dimethylformamid, Dichlormethan oder Diethylglykolmonoethylether umfasst; wobei das Lösungsmittel gegebenenfalls durch C₈-C₁₀-Capryl/Caprintriglycerid, Oleinsäure oder Propylenglykol ergänzt ist; und
das organische Colösungsmittel ausgewählt ist aus der Gruppe, bestehend aus Ethanol, Isopropanol und Methanol;
für die Verwendung bei der Bekämpfung von Parasiten einer Katze oder eines Hundes, umfassend die lokalisierte kutane Applikation auf die Katze oder den Hund zwischen den Schultern mit einer Frequenz nicht größer als monatlich, wobei eine verlängerte Freisetzung der Verbindung (A) in oder auf dem Körper der Katze oder des Hundes gegeben ist und ein messbarer Plasmaspiegel der Verbindung (B) in der Katze oder dem Hund vorliegt.

14. Die Spot-on-Zusammensetzung zur Verwendung gemäß Anspruch 13, wobei die Verbindung (A) 1-[4-CF₃-2,6-Cl₂-phenyl]-3-cyano-4-[CF₃-SO]-5-NH₂-pyrazol ist.

15. Die Spot-on-Zusammensetzung zur Verwendung gemäß Anspruch 14, wobei Verbindung (A) in einer Menge von 0,1 bis 100 mg/kg des Tiergewichtes vorliegt und Verbindung (B) in der Spot-on-Zusammensetzung in einer Menge von 1 µg bis 1 mg/kg des Tiergewichtes vorliegt.

16. Die Spot-on-Zusammensetzung zur Verwendung gemäß Anspruch 13, wobei der Kristallisationsinhibitor ein Kristallisationsinhibitorpaar ist.

17. Die Spot-on-Zusammensetzung gemäß Anspruch 13, wobei das organische Lösungsmittel Diethylenglykolmonoethylether ist und das organische Colösungsmittel Ethanol oder Isopropanol ist.

18. Die Spot-on-Zusammensetzung zur Verwendung gemäß Anspruch 14, wobei ein Verhältnis von Gewicht/Gewicht des Colösungsmittels/Lösungsmittels vorliegt und das Verhältnis von Gewicht/Gewicht des Colösungsmittels/Lösungsmittels zwischen 1/15 und 1/2 beträgt.

19. Die Spot-on-Zusammensetzung zur Verwendung gemäß Anspruch 14, ferner umfassend Wasser in einer Menge von weniger als 30% (Vol./Vol.).

20. Die Spot-on-Zusammensetzung zur Verwendung gemäß Anspruch 14, wobei die Spot-on-Zusammensetzung kein Wasser enthält.

21. Die Spot-on-Zusammensetzung zur Verwendung gemäß Anspruch 14, ferner umfassend ein Antioxidans.

22. Die Spot-on-Zusammensetzung zur Verwendung gemäß Anspruch 21, wobei das Antioxidans in einer Menge von 0,005 bis 1% (Gew./Vol.) vorliegt und ausgewählt ist aus der Gruppe, bestehend aus butyliertem Hydroxyanisol, butyliertem Hydroxytoluol, Ascorbinsäure, Natriummetabisulfit, Propylgallat und Natriumthiosulfat.

23. Verwendung einer Spot-on-Zusammensetzung, die eine parasitär wirksame Menge von mindestens einer Verbindung (A) und eine parasitär wirksame Menge von mindestens einer Verbindung (B) umfasst, wobei:
Verbindung (A) die Formel (I) aufweist 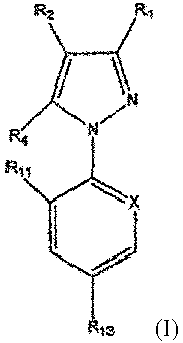
in der:
R₁ CN ist;
R₂ S(O)ₙR₃ ist;
R₃ Halogenalkyl ist;
R₄ NH₂ darstellt;
R₁₁ Halogenatom darstellt;
R₁₃ Halogenalkyl darstellt;
n eine ganze Zahl gleich 0, 1 oder 2 darstellt;
X einen Rest C-R₁₂ darstellt;
R₁₂ ein Halogenatom darstellt;
und
Verbindung (B) ein endektizides Parasitizid ist, das Emamectin oder ein Salz davon ist,
zur Herstellung eines Medikaments, dass einen veterinärmedizinisch verträglichen Träger zur Bekämpfung von Parasiten bei einer Katze oder einem Hund umfasst, umfassend die lokalisierte kutane Applikation auf die Katze oder den Hund zwischen den Schultern mit einer Frequenz nicht größer als monatlich, wobei der Träger für eine lokalisierte kutane Applikation auf das Tier zwischen den Schultern ist und ein organisches Lösungsmittel,
ein organisches Colösungsmittel und/oder einen Kristallisationsinhibitor enthält, wobei:
der Kristallisationsinhibitor ausgewählt ist aus der Gruppe bestehend aus Polyvinylpyrrolidon, Copolymeren von Vinylacetat und Vinylpyrrolidon, polyoxyethylenierten Sorbitanestern und Mischungen davon;
das organische Lösungsmittel Aceton, Ethylacetat, Methanol, Ethanol, Isopropanol, Dimethylformamid, Dicholormethan oder Diethylglykolmonoethylether umfasst; wobei das Lösungsmittel gegebenenfalls durch C₈-C₁₀-Capryl/Caprintriglycerid, Oleinsäure oder Propylenglykol ergänzt ist; und
das organische Colösungsmittel ausgewählt ist aus der Gruppe, bestehend aus Ethanol, Isopropanol und Methanol;
wobei eine verlängerte Freisetzung der Verbindung (A) in oder auf dem Körper der Katze oder des Hundes gegeben ist und ein messbarer Plasmaspiegel der Verbindung (B) in der Katze oder dem Hund vorliegt.

24. Die Verwendung gemäß Anspruch 23, wobei Verbindung (A) 1-[4-CF₃-2,6-Cl₂-phenyl]-3-cyano-4-[CF₃-SO]-5-NH₂-pyrazol ist.

## Revendications

1. Formulation de type spot-on pour le traitement ou la prophylaxie d'une infestation parasitaire chez des mammifères ou des oiseaux qui comprend
(1) une composition comprenant
(A) une quantité efficace de (1) 1-[2,6-Cl₂-4-CF₃ phényl]-3-CN-4-[SO-CF₃]-5-NH₂ pyrazole (fipronil) ; et
(B) une quantité efficace d'émamectine ou d'un sel de celle-ci ;
(2) un véhicule vecteur liquide acceptable sur le plan pharmaceutique ou vétérinaire, dans lequel le véhicule vecteur liquide comprend un solvant et un co-solvant dans lequel le solvant est choisi dans le groupe consistant en l'acétone, l'acétonitrile, l'alcool benzylique, le butyl diglycol, le diméthylacétamide, le diméthylformamide, le dipropylène glycol n-butyl éther, l'éthanol, l'isopropanol, le méthanol, l'éthylène glycol monoéthyl éther, l'éthylène glycol monométhyl éther, le monométhylacéamide, le dipropylène glycol monométhyl éther, les polyoxyéthylène glycol liquides, le propylène glycol, la 2-pyrrolidone, le diéthylène glycol monoéthyl éther, l'éthylène glycol, le phtalate de diéthyle, et un mélange d'au moins deux de ces solvants et le co-solvant est choisi dans le groupe consistant en l'éthanol absolu, l'isopropanol ou le méthanol ;
(3) un inhibiteur de cristallisation, dans lequel l'inhibiteur de cristallisation est choisi dans le groupe consistant en un tensioactif anionique, un tensioactif cationique, un tensioactif non ionique, un sel d'amine, un tensioactif amphotère, la polyvinylpyrrolidone, les poly(alcools vinyliques), les copolymères d'acétate de vinyle et de vinylpyrrolidone, les poly(éthylènes glycol), l'alcool benzylique, le mannitol, le glycérol, le sorbitol, les esters de sorbitan polyoxyéthylénés ; la lécithine, la carboxyméthylcellulose de sodium, et les dérivés acryliques, ou un mélange de ces inhibiteurs de cristallisation.

2. Formulation de type spot-on selon la revendication 1, dans laquelle le véhicule vecteur liquide comprend une microémulsion.

3. Formulation de type spot-on selon la revendication 1, dans laquelle le véhicule vecteur liquide comprend en outre un diluant.

4. Formulation de type spot-on selon la revendication 1, dans laquelle la combinaison comprend 0,001 à 100 mg/kg de poids de mammifère ou d'oiseau de (1) 1-[2,6-Cl₂-4-CF₃ phényl]-3-CN-4-[SO-CF₃]-5-NH₂ pyrazole et entre 0,1 mg à 10 mg/kg de poids de mammifère ou d'oiseau d'émamectine ou d'un sel de celle-ci.

5. Formulation de type spot-on selon la revendication 1, dans laquelle la combinaison comprend 1 à 50 mg/kg de poids de mammifère ou d'oiseau de (1) 1-[2,6-Cl₂-4-CF₃ phényl]-3-CN-4-[SO-CF₃]-5-NH₂ pyrazole et entre 0,1 µg et 1 mg/kg par poids de mammifère ou d'oiseau d'émamectine ou d'un sel de celle-ci.

6. Formulation de type spot-on selon la revendication 1, dans laquelle la combinaison comprend entre 5 et 500 µg/kg par poids de mammifère ou d'oiseau d'émamectine ou d'un sel de celle-ci.

7. Formulation de type spot-on selon la revendication 4, qui comprend 0,5 mg/kg d'émamectine par poids de mammifère ou d'oiseau.

8. Formulation de type spot-on selon la revendication 1, dans laquelle la combinaison comprend le rapport, en poids, de (1) 1-[2,6-Cl₂-4-CF₃ phényl]-3-CN-4-[SO-CF₃]-5-NH₂ pyrazole sur l'émamectine ou un sel de celle-ci de 5/1 à 10 000/1.

9. Formulation de type spot-on selon la revendication 1, qui comprend en outre un antioxydant dans lequel 0,005 à 1 % (p/v) d'antioxydant est présent et l'antioxydant est choisi dans le groupe consistant en l'hydroxyanisole butylé, l'hydroxytoluène butylé, l'acide ascorbique, le métabisulfite de sodium, le gallate de propyle, et le thiosulfate de sodium.

10. Formulation de type spot-on selon la revendication 1, qui comprend en outre de l'eau, dans laquelle l'eau est présente dans une proportion de 0 à 30 % (v/v).

11. Formulation de type spot-on selon la revendication 1, dans laquelle l'inhibiteur de cristallisation est présent dans une quantité de 1 à 20 % (p/v) et dans laquelle l'inhibiteur de cristallisation est un système inhibiteur de cristallisation comprenant un agent filmogène polymérique et un tensioactif, dans laquelle l'agent filmogène polymérique est la polyvinylpyrrolidone, des poly(alcools vinyliques), ou un copolymère d'acétate de vinyle et de polyvinylpyrrolidone et le tensioactif est un tensioactif non ionique.

12. Formulation de type spot-on selon la revendication 1, dans laquelle
- le tensioactif anionique est des stéarates alcalins, l'abiétate de sodium ; des sulfates d'alkyle ; le dodécylbenzènesulfonate de sodium, le dioctylsulfosuccinate de sodium ; et des acides gras ;
- le tensioactif cationique est des sels d'ammonium quaternaire solubles dans l'eau de formule N⁺R'R"R"'R""Y⁻ dans lequel les radicaux R sont indépendamment des radicaux hydrocarbures, facultativement hydroxylés, et Y⁻ est un anion d'un acide fort ;
- le sel d'amine est un sel d'amine de N⁺R'R"R'" dans lequel les radicaux R sont indépendamment des radicaux hydrocarbures facultativement hydroxylés ;
- le tensioactif non ionique est facultativement des esters de sorbitan polyoxyéthylénés, des alkyl éther polyoxyéthylénés ; le stéarate de poly(éthylène glycol), les dérivés polyoxyéthylénés d'huile de ricin, les esters de polyglycérol, les alcools gras polyoxyéthylénés, les acides gras polyoxyéthylénés, les copolymères d'oxyde d'éthylène et d'oxyde de propylène ; et
- le tensioactif amphotère est des composés de bétaïne substitués par lauryle.

13. Composition de type spot-on, qui comprend, dans un véhicule acceptable sur le plan vétérinaire, une quantité efficace sur le plan parasitaire d'au moins un composé (A), et une quantité efficace sur le plan parasitaire d'au moins un composé (B), dans laquelle :
le composé (A) est de formule (I) 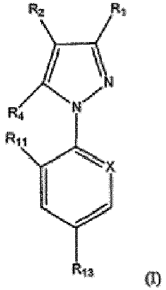
dans laquelle :
R₁ est CN ;
R₂ est S(O)ₙR₃ ;
R₃ est halogénoalkyle ;
R₄ représente NH₂ ;
R₁₁ représente un atome d'halogène ;
R₁₃ représente halogénoalkyle ;
n représente un entier égal à 0, 1 ou 2 ;
X représente un radical C-R₁₂ ;
R₁₂ représente un atome d'halogène ;
et
le composé (B) est un parasiticide endectocide qui est l'émamectine, ou un sel de celle-ci ; et,
le véhicule est destiné à une application cutanée localisée à l'animal entre les épaules et contient un solvant organique, un co-solvant organique et/ou un inhibiteur de cristallisation dans laquelle :
l'inhibiteur de cristallisation est choisi dans le groupe consistant en la polyvinylpyrrolidone, les copolymères d'acétate de vinyle et de vinylpyrrolidone, les esters de sorbitan polyoxyéthylénés et leurs mélanges ;
le solvant organique comprend l'acétone, l'acétate d'éthyle, le méthanol, l'éthanol, l'isopropanol, le diméthylformamide, le dichlorométhane ou le diéthyl glycol monoéthyl éther ; ledit solvant étant facultativement complémenté par un triglycéride caprylique/caprique en C₈ à C₁₀, l'acide oléique ou le propylène glycol ;
et
le co-solvant organique est choisi dans le groupe consistant en l'éthanol, l'isopropanol, et le méthanol ;
à utiliser dans la lutte contre des parasites d'un chat ou d'un chien comprenant l'application cutanée localisée au chat ou au chien, entre les épaules, à une fréquence de pas plus d'un mois, moyennant quoi on a une libération prolongée du composé (A) dans ou sur le corps du chat ou du chien et on a un niveau plasmatique mesurable du composé (B) chez le chat ou le chien.

14. Composition de type spot-on à utiliser selon la revendication 13, dans laquelle le composé (A) est le 1-[4-CF₃ 2,6-Cl₂ phényl]3-cyano 4-[CF₃-SO]5-NH₂ pyrazole.

15. Composition de type spot-on à utiliser selon la revendication 14, dans laquelle le composé (A) est présent dans une quantité de 0,1 à 100 mg/kg de poids d'animal et le composé (B) est présent dans la composition de type spot-on dans une quantité de 1 µg à 1 mg/kg de poids d'animal.

16. Composition de type spot-on à utiliser selon la revendication 13, dans laquelle l'inhibiteur de cristallisation est une paire d'inhibiteurs de cristallisation.

17. Composition de type spot-on selon la revendication 13, dans laquelle le solvant organique est le diéthylène glycol monoéthyl éther et le co-solvant organique est l'éthanol ou l'isopropanol.

18. Composition de type spot-on à utiliser selon la revendication 14, dans laquelle on a un rapport poids/poids du co-solvant/solvant et le rapport poids/poids du co-solvant/solvant se situe entre 1/15 et 1/2.

19. Composition de type spot-on à utiliser selon la revendication 14, comprenant en outre de l'eau dans une quantité de moins de 30 % (v/v).

20. Composition de type spot-on à utiliser selon la revendication 14, dans laquelle la composition de type spot-on ne contient pas d'eau.

21. Composition de type spot-on à utiliser selon la revendication 14, comprenant en outre un antioxydant.

22. Composition de type spot-on à utiliser selon la revendication 21, dans laquelle l'antioxydant est présent dans une quantité de 0,005 à 1 % (p/v) et est choisi dans le groupe consistant en l'hydroxyanisole butylé, l'hydroxytoluène butylé, l'acide ascorbique, le métabisulfite de sodium, le gallate de propyle, et le thiosulfate de sodium.

23. Utilisation d'une composition de type spot-on, qui comprend, une quantité efficace sur le plan parasitaire d'au moins un composé (A), et une quantité efficace sur le plan parasitaire d'au moins un composé (B), dans laquelle :
le composé (A) est de formule (I) 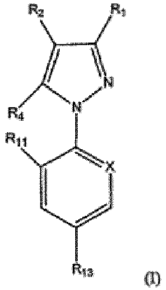
dans laquelle :
R₁ est CN ;
R₂ est S(O)ₙR₃ ;
R₃ est halogénoalkyle ;
R₄ représente NH₂ ;
R₁₁ représente un atome d'halogène ;
R₁₃ représente halogénoalkyle ;
n représente un entier égal à 0, 1 ou 2 ;
X représente un radical C-R₁₂ ;
R₁₂ représente un atome d'halogène ;
et
le composé (B) est un parasiticide endectocide qui est l'émamectine, ou un sel de celle-ci ; et,
pour la fabrication d'un médicament qui comprend un véhicule acceptable sur le plan vétérinaire destiné à lutter contre des parasites d'un chat ou d'un chien comprenant une application cutanée localisée au chat ou au chien, entre les épaules, à une fréquence de pas plus d'une fréquence mensuelle, dans lequel le véhicule est destiné à une application cutanée localisée à l'animal entre les épaules et contient un solvant organique, un co-solvant organique et/ou un inhibiteur de cristallisation dans lequel :
l'inhibiteur de cristallisation est choisi dans le groupe consistant en la polyvinylpyrrolidone, les copolymères d'acétate de vinyle et de vinylpyrrolidone, les esters de sorbitan polyoxyéthylénés et leurs mélanges ;
le solvant organique comprend l'acétone, l'acétate d'éthyle, le méthanol, l'éthanol, l'isopropanol, le diméthylformamide, le dichlorométhane ou le diéthyl glycol monoéthyl éther ; ledit solvant étant facultativement complémenté par un triglycéride caprylique/caprique en C₈ à C₁₀, l'acide oléique ou le propylène glycol ;
et
le co-solvant organique est choisi dans le groupe consistant en l'éthanol, l'isopropanol, et le méthanol ;
moyennant quoi on a une libération prolongée de composé (A) dans ou sur le corps du chat ou du chien et on a un niveau plasmatique mesurable du composé (B) chez le chat ou le chien.

24. Utilisation selon la revendication 23, dans laquelle le composé (A) est le 1-[4-CF₃ 2,6-Cl₂ phényl] 3-cyano 4-[CF₃-SO] 5-NH₂ pyrazole.
